# EUROPÄISCHE PATENTSCHRIFT

(11) **EP 1 485 047 B1**
(45) Veröffentlichungstag und Bekanntmachung des Hinweises auf die Patenterteilung: **07.06.2006**
(21) Anmeldenummer: 03714795.6
(22) Anmeldetag: 10.03.2003
(51) Int. Cl.: A61F 5/02

(54) **STÜTZRAHMEN ZUR ENTLASTUNG DER WIRBELSÄULE**
SUPPORT FRAME FOR RELIEVING THE VERTEBRAL COLUMN
CORSET DESTINE A SOULAGER LA COLONNE VERTEBRALE

(30) Priorität: 12.03.2002 DE 10210775; 12.03.2002 DE 20204747 U
(43) Veröffentlichungstag der Anmeldung: 15.12.2004
(73) Patentinhaber: Zours, Claudia, 44797 Bochum (DE)
(72) Erfinder: ZOURS, Claudia, 44797 Bochum (DE); KOPPETSCH, Gerd-Peter, 40627 Düsseldorf (DE); KRÄMER, Robert, 40213 Düsseldorf (DE)
(74) Vertreter: Schneiders, Josef
(86) Internationale Anmeldenummer: PCT/EP2003/002430
(87) Internationale Veröffentlichungsnummer: WO 2003/075807

(56) Entgegenhaltungen:
- DE-U- 8 907 580
- GB-A- 2 215 607
- US-A- 2 835 247
- US-A- 3 889 664
- US-A- 5 362 304
- LEONHARDT R.: 'Neuartige Wirbelsäulenorthesen mit Stufentherapieplan aus Sicht des niedergelassenen Orthopäden', April 2003, MEDIZINISCH LITERARISCHE VERLAGSGESELLSCHAFT MBH, UELZEN, SONDERDRUCK ORTHOPCDISCHE PRAXIS 4/2003, 39. JAHRGANG
- DR. KRÄMER R.; KOPPETSCH G.P.: 'Therapeutisch korrekte Abschulung von Wirbelsäulen-Orthesen', 2003, MOT, ORGAN DER ISPO = INTERNATIONAL SOCIETY FOR PROTHETICS AND ORTHOTICS, SONDERDRUCK AUS MEDIZINISCH-OHOPCDISCHE TECHNIK, HEFT 1, 2003

## Beschreibung

Die Erfindung betrifft einen Stützrahmen zur Entlastung der Wirbelsäule im Rumpfbereich zwischen Hüfte und Brustwirbelsäule, mit einer sich auf der Hüfte des Patienten abstützenden Hüftspange und einer den Brustwirbelbereich abstützenden BWS-Spange, die durch parallel zur Wirbelsäule verlaufende Stützstäbe miteinander verbunden sind, wobei der Abstand zwischen der Hüftspange und der BWS-Spange dadurch verstellbar ist, daß die parallel zur Wirbelsäule verlaufenden Stütztstäbe längenveränderlich sind.

Solche Stützrahmen sind aus der Druckschrift DE-U-89 07 580 bekannt und werden beispielsweise in Verbindung mit Wirbelsäulenorthesen oder sogenannten Überbrückungsmiedern für die postoperative Behandlung von Patienten mit Wirbelsäulenleiden verwendet.

Für die Anpassung des Stützrahmens an die Größe von Patienten reicht es aus, mit Hilfe der längenveränderlichen Stützstäbe, den Abstand zwischen Hüftspange und BWS-Spange zu verstellen. Die Hüftspange und die BWS-Spange selbst können demgegenüber aufgrund ihrer Verformbarkeit ohne weiteres an unterschiedliche Körperformen, insbesondere an den Körperumfang von Patienten angepasst werden. Somit kann der Stützrahmen gemäß der Erfindung insgesamt ohne weiteres an alle individuellen Körpermaße und Körperformen des Patienten angepasst werden. Für die fachgerechte Versorgung von Patienten reicht es deshalb aus, nur wenige Stützrahmengrößen bereitzuhalten.

Vergleichbare Vorrichtungen mit der Möglichkeit zur Einstellung des Abstandes zwischen einem Hüftgürtel und den Oberkörper umgreifenden Gürteln oder Bögen sind grundsätzlich z. B. aus der US-3 889 664 oder US- 2 835 247 bekannt.

Es ist Aufgabe der Erfindung, den genannten Stützrahmen der genannten Art auf einfache Weise den Bedürfnissen des Patienten entsprechend starrer oder flexibler zu gestalten, d. h. dem Stützrahmen die jeweils geforderte Steifigkeit zu geben.

Diese Aufgabe wird durch die Merkmale des Patentanspruchs 1 gelöst.

Mit Hilfe der erfindungsgemäß zusätzlich vorgesehenen austauschbaren Versteifungsstäbe unterschiedlicher Steifigkeit ist es auf einfache Weise möglich, die Steifigkeit des Stützrahmens dem jeweiligen Bedarf entsprechend zu beeinflussen, d. h. den Stützrahmen starrer oder flexibler zu machen.

Die längenveränderlichen Stützstäbe des Stützrahmens sind zweckmäßig unterteilt ausgebildet, wobei die Stützstabteile sich in Längsrichtung der Wirbelsäule gesehen überlappen und im Überlappungsbereich verstellbar aneinander festlegbar sind. Die Stützstabteile sind in diesem Fall fest, ggf. einstückig mit der Hüftspange bzw. der BWS-Spange verbunden und im Überlappungsbereich mit Befestigungsmitteln versehen, mit denen sie aneinander festgelegt werden können.

Besondere Vorteile ergeben sich, wenn die Stützstabteile im Überlappungsbereich durch Klettverschlüsse aneinander festlegbar sind. Solche Klettverschlüsse haben den Vorteil, dass sie besonders einfach handhabbar sind und in Längsrichtung der Stützstäbe ausreichend große Kräfte übertragen können, wobei die Stützstabteile praktisch stufenlos gegeneinander verstellt werden können.

Anstelle der Klettverschlüsse können auch Druckknöpfe, Klebestreifen oder Haken und Ösen verwendet werden.

Gegebenenfalls können die Stützstabteile im Überlappungsbereich auch teleskopartig aneinander geführt sein und in unterschiedlichen Ausfahrlängen aneinander fixierbar sein. Auch diese teleskopartig aneinander geführten Stützstabteile sind zweckmäßig fest ggf. einstückig mit der Hüftspange bzw. der BWS-Spange verbunden.

Die parallel zur Wirbelsäule verlaufenden Versteifungsstäbe sind sowohl an der Hüftspange als auch an der BWS-Spange lösbar befestigt und gegen Versteifungsstäbe mit einer anderen Länge und Steifigkeit austauschbar.. Die austauschbaren Versteifungsstäbe können z. B. durch Klettverbindungen, Druckknöpfe, Klebeverbindungen, Haken und Ösen oder dergleichen an der Hüftspange einerseits und der BWS-Spange andererseits festgelegt werden.

Vorzugsweise ist jedoch vorgesehen, dass an der Hüftspange einerseits und der BWS-Spange andererseits Aufnahmetaschen vorgesehen sind, in die Versteifungsstäbe unterschiedlicher Länge einsetzbar sind. In diesem Fall sind die Versteifungsstäbe natürlich nicht Bestandteil der Hüftspange bzw. der BWS-Spange, sondern von diesen getrennte Teile. Für die Anpassung der Stützweite des Stützrahmens müssen in diesem Fall unterschiedlich lange Versteifungsstäbe vorrätig gehalten werden und den Körpermaßen des Patienten entsprechend ausgewählt werden. Alternativ kann man die auswechselbaren Versteifungsstäbe auch so ausbilden, dass sie mit einfachen Hilfsmitteln, z. B. mit Hilfe eines geeigneten Schneidwerkzeugs, auf die jeweils richtige Länge eingekürzt werden können.

Die Versteifungsstäbe können aus einem geeigneten, steifen Kunststoff und/oder aus Stahl bestehen. Solche mit Stahl verstärkten oder gänzlich aus Stahl bestehenden Versteifungsstäbe haben eine besonders hohe Steifigkeit bei geringem Gewicht und geringen äußerlichen Abmessungen. Letzteres ist besonders wichtig, damit der Stützrahmen, der gegebenenfalls unter der Kleidung getragen wird, nicht zu sehr aufträgt.

Für den Fall, dass der Stützrahmen gemäß der Erfindung zusätzlich zur Entlastung der Brustwirbelsäule verwendet werden soll, ist weiterhin vorgesehen, dass die BWS-Spange zusätzlich mit einer bis unter die Schulterblätter des Patienten reichenden Verlängerung versehen ist, die ebenfalls von den in diesem Fall entsprechend länger ausgebildeten Stützstäben gehalten wird. Mit einer solchen Verlängerung der BWS-Spange ist es möglich, zusätzlich zum Lumbalbereich auch den Brustwirbelbereich der Wirbelsäule zu unterstützen.

Ausführungsbeispiele der Erfindung werden im folgenden anhand der Zeichnungen näher erläutert. Es zeigen:
- Figur 1:: Einen Stützrahmen gemäß der Erfindung in einer ersten Ausführungsform;
- Figur 2:: Einen Stützrahmen gemäß der Erfindung in einer zweiten Ausführungsform.

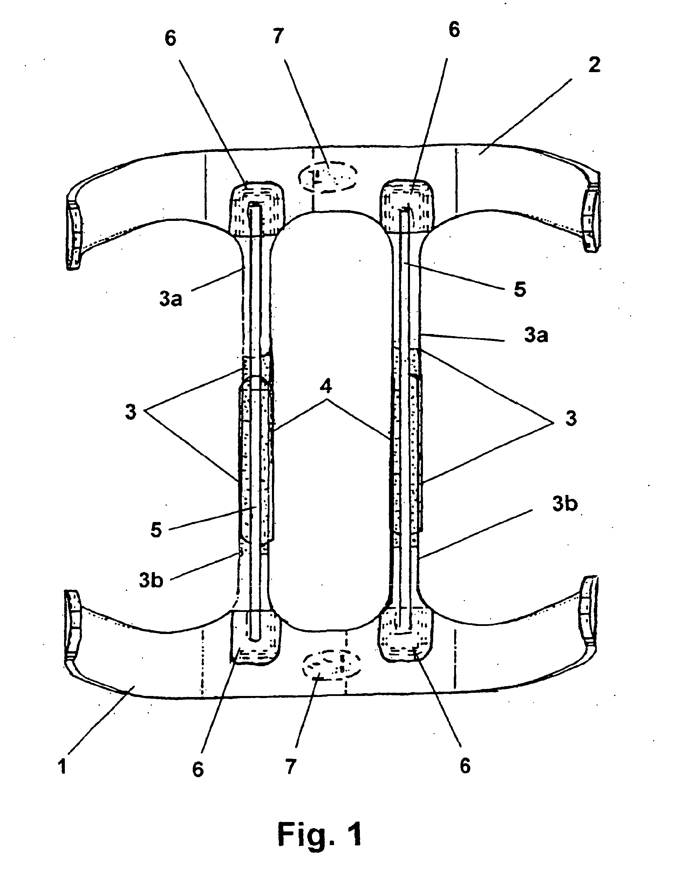

Der in Figur 1 dargestellte Stützrahmen weist eine sich auf der Hüfte des Patienten abstützende Hüftspange 1 und eine den Rücken im Bereich der Brustwirbelsäule abstützende BWS-Spange 2 auf, die durch parallel zur Wirbelsäule verlaufende Stützstäbe 3 miteinander verbunden sind. Diese Stützstäbe 3 des Stützrahmens sind jeweils unterteilt, wobei die Stützstababschnitte 3a und 3b sich in Längsrichtung der Wirbelsäule gesehen überlappen und im Überlappungsbereich mittels Klettverschlüssen 4 aneinander festlegbar sind. Auf diese Weise sind die Stützstäbe 3 längenveränderlich und damit die Stützweite des Stützrahmens verstellbar.

Zur zusätzlichen Versteifung oder ggf. als Ersatz für die oben erwähnten Stützstäbe 3 sind zusätzlich austauschbare Versteifungsstäbe 5 vorgesehen, die beispielsweise aus Stahl bestehen können und in Aufnahmetaschen 6 einsteckbar sind, die sich einerseits an der Hüftspange 1 und andererseits an der BWS-Spange 2 befinden. Die austauschbaren Versteifungsstäbe 5 können ggf. auch mit anderen lösbaren Befestigungsmitteln an der Hüftspange 1 einerseits und der BWS-Spange 2 andererseits befestigt werden.

Der gesamte Rückenstützrahmen besteht aus thermoplastischem Kunststoff, so dass er durch Erwärmen plastisch verformt werden kann, um ihn der Anatomie des Patienten anpassen zu können. Alternativ wäre auch ein anderes, verformbares Material denkbar.

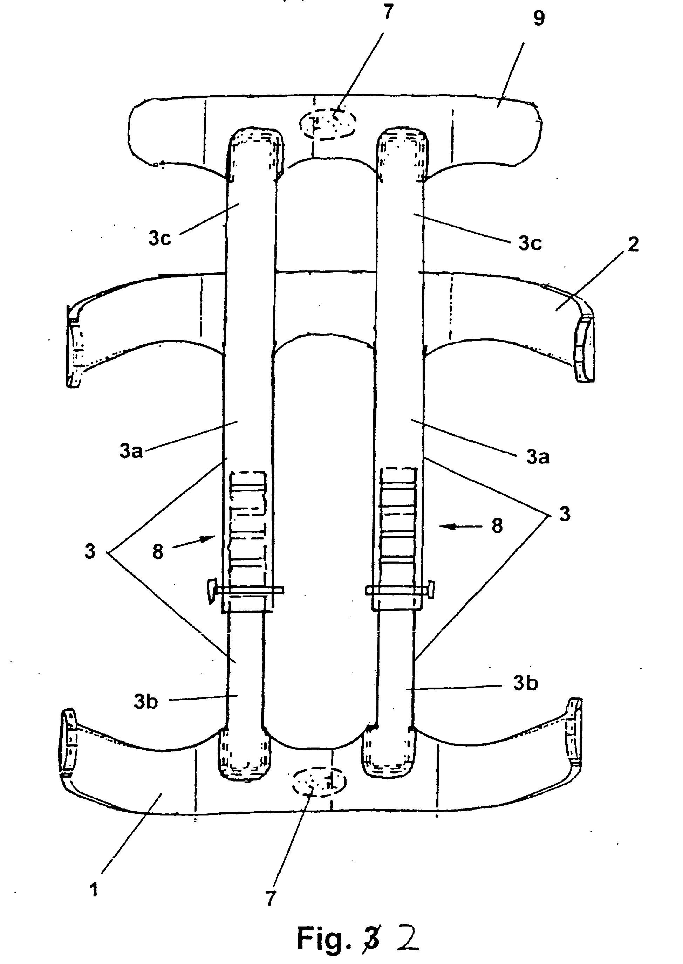

Beim Ausführungsbeispiel gemäß Figur 2, bei dem die zur Erfindung gehörenden austauschbaren Versteifungsstäbe nicht eingezeichnet sind, sind die einstückig mit der Hüftspange 1 bzw. der BWS-Spange 2 verbundenen Stützstababschnitte 3a und 3b teleskopartig aneinander geführt und in unterschiedlichen Ausfahrlängen aneinander fixierbar. Letzteres geschieht bei dieser Ausführungsform durch die mit 8 bezeichnete Loch-Steckverbindung.

Außerdem sind bei diesem Ausführungsbeispiel die Stützstäbe 3 über die BWS-Spange 2 hinaus durch Stützstababschnitte 3c verlängert, welche ein unterhalb der Schulterblätter des Patienten angeordnetes Abstützelement 9 tragen. Dieses Abstützelement 9 stützt die Wirbelsäule oberhalb der BWS-Spange im Bereich der Brustwirbelsäule. Dieses Abstützelement 9 wäre auch bei den Ausführungsbeispielen 1 und 2 denkbar.

Bei allen drei Ausführungsbeispielen sind die Hüftspange 1 und/oder die BWS-Spange 2 weiterhin mit Klettverschlüssen 7 versehen, mit denen der Rückenstützrahmen an Bandagen oder Überbrückungsmiedern festgelegt werden kann, die in der Zeichnung nicht dargestellt sind.

## Patentansprüche

1. Stützrahmen zur Entlastung der Wirbelsäule im Rumpfbereich zwischen Hüfte und Brustwirbelsäule, mit einer sich auf der Hüfte des Patienten abstützenden Hüftspange (1) und einer den Brustwirbelbereich abstützenden BWS-Spange (2), die durch parallel zur Wirbelsäule verlaufende Stützstäbe (3) miteinander verbunden sind, wobei der Abstand zwischen der Hüftspange (1) und der BWS-Spange (2) dadurch verstellbar ist, daß die parallel zur Wirbelsäule verlaufenden Stützstäbe (3) längenveränderlich sind, **dadurch gekennzeichnet, daß** der Stützrahmen zusatzlich austauschbare Versteifungsstäbe (5) unterschiedlicher Steifigkeit aufweist, und daß diese versteitungsstäbe (5) zwischen die Hüftspange (1) und die BWS-Spange (2) einsetzbar sind

2. Stützrahmen nach Anspruch 1, **dadurch gekennzeichnet, daß** die austauschbaren Versteifungsstäbe (5) aus Kunststoff und/oder aus Stahl bestehen.

3. Stützrahmen nach den Ansprüchen 1 und 2, **dadurch gekennzeichnet, daß** an der Hüftspange (1) einerseits und der BWS-Spange (2) andererseits Aufnahmetaschen (6) vorgesehen sind, in die die austauschbaren Versteifungsstäbe (5) einsetzbar sind.

4. Stützrahmen nach einem der Ansprüche 1 bis 3, **dadurch gekennzeichnet, daß** die längenveränderlichen Stützstäbe (3) des Stützrahmens jeweils unterteilt sind, wobei die Stützstabteile (3a, 3b) sich in Längsrichtung der Wirbelsäule gesehen überlappen und im Überlappungsbereich verstellbar aneinander festlegbar sind.

5. Stützrahmen nach Anspruch 4, **dadurch gekennzeichnet, daß** die Stützstabteile (3a, 3b) einstückig mit der Hüftspange (1) bzw. der BWS-Spange (2) verbunden sind und im Überlappungsbereich mit Befestigungsmitteln versehen sind, mit denen sie aneinander festlegbar sind.

6. Stützrahmen nach Anspruch 5, **dadurch gekennzeichnet, daß** die Stützstabteile (3a, 3b) im Überlappungsbereich durch Klettverschlüsse (4) aneinander festlegbar sind.

7. Stützrahmen nach einem der Ansprüche 1 bis 4, **dadurch gekennzeichnet, daß** die Stützstabteile (3a, 3b) teleskopartig aneinander geführt und in unterschiedlichen Ausfahrlängen aneinander fixierbar sind.

8. Stützrahmen nach einem der Ansprüche 1 bis 7, **dadurch gekennzeichnet, daß** die BWS-Spange (2) zusätzlich mit einer bis unter die Schulterblätter des Patienten reichenden Verlängerung (3c, 9) versehen ist, die ebenfalls von den in diesem Fall entsprechend länger ausgebildeten Stützstäben (3) gehalten wird.

## Claims

1. A support frame for relieving the load on the spinal column in the trunk region between the hip and the thoracic spinal column, comprising a hip brace (1) supported on the hip of the patient and a TSC brace (2) supporting the thoracic spinal column region, the braces being connected together by support bars (3) extending parallel to the spinal column, wherein the spacing between the hip brace (1) and the TSC brace (2) is adjustable by the support bars (3) extending parallel to the spinal column being variable in length, **characterised in that** the support frame additionally has interchangeable stiffening bars (5) of different stiffness and that said stiffening bars (5) can be fitted between the hip brace (1) and the TSC brace (2).

2. A support frame according to claim 1 **characterised in that** the interchangeable stiffening bars (5) comprise plastic material and/or steel.

3. A support frame according to claims 1 and 2 **characterised in that** provided on the hip brace (1) on the one hand and the TSC brace (2) on the other hand are receiving pockets (5) into which the interchangeable stiffening bars (5) can be fitted.

4. A support frame according to one of claims 1 to 3 **characterised in that** the variable-length support bars (3) of the support frame are each subdivided, wherein the support bar portions (3a, 3b) mutually overlap as viewed in the longitudinal direction of the spinal column and can be fixed adjustably to each other in the overlap region.

5. A support frame according to claim 4 **characterised in that** the support bar portions (3a, 3b) are integrally connected to the hip brace (1) and the TSC brace (2) respectively and in the overlap region are provided with securing means with which they can be fixed to each other.

6. A support frame according to claim 5 **characterised in that** the support bar portions (3a, 3b) can be fixed to each other in the overlap region by hook-and-loop fasteners (4).

7. A support frame according to one of claims 1 to 4 **characterised in that** the support bar portions (3a, 3b) are guided telescopically on each other and can be fixed to each other at different extension lengths.

8. A support frame according to one of claims 1 to 7 **characterised in that** the TSC brace (2) is additionally provided with an extension (3c, 9) which extends to below the shoulder blades of the patient and which is also held by the support bars (3) which in this case are correspondingly longer.

## Revendications

1. Corset destiné à soulager la colonne vertébrale dans la zone du tronc entre la hanche et les vertèbres dorsales, avec une boucle de hanche (1) s'appuyant sur la hanche du patient et une boucle BWS (2) soutenant la zone des vertèbres dorsales, qui sont reliées entre elles par des tiges de support (3) s'étendant parallèlement à la colonne vertébrale, où l'écart entre la boucle de hanche (1) et la boucle BWS (2) est ajustable en ce que la longueur des tiges de support (3) s'étendant parallèlement à la colonne vertébrale peut être modifiée, **caractérisé en ce que** le corset présente de plus des tiges de renforcement échangeables (5) d'une rigidité différente, et **en ce que** ces tiges de renforcement (5) peuvent être insérées entre la boucle de hanche (1) et la boucle BWS (2).

2. Corset selon la revendication 1, **caractérisé en ce que** les tiges de renforcement échangeables (5) sont en matériau synthétique et/ou en acier.

3. Corset selon la revendications 1 et 2, **caractérisé en ce que** sont prévues à la boucle de hanche (1), d'une part et à la boucle BWS (2) d'autre part des poches de réception (6) dans lesquelles peuvent être placées les tiges de renforcement échangeables (5).

4. Corset selon l'une des revendications 1 à 3, **caractérisé en ce que** les tiges de support (3) à longueur modifiable du corset sont à chaque fois subdivisées, où les parties de tige de support (3a,3b), en regardant dans la direction longitudinale de la colonne vertébrale, se chevauchent et peuvent être fixées d'une manière ajustable l'une à l'autre dans la zone de chevauchement.

5. Corset selon la revendication 4, **caractérisé en ce que** les parties de tige de support (3a,3b) sont reliées en une pièce avec la boucle de hanche (1) respectivement la boucle BWS (2) et présentent dans la zone de chevauchement des moyens de fixation par lesquels elles peuvent être fixées l'une à l'autre.

6. Corset selon la revendication 5, **caractérisé en ce que** les parties de tige de support (3a,3b) peuvent être fixées l'une à l'autre dans la zone de chevauchement par des fermetures auto-accrochantes (4).

7. Corset selon l'une des revendications 1 à 4, **caractérisé en ce que** les parties de tige de support (3a,3b) sont guidées d'une manière télescopique l'une à l'autre et peuvent être fixées l'une à l'autre à différentes longueurs de sortie.

8. Corset selon l'une des revendications 1 à 7, **caractérisé en ce que** le corset BWS (2) est muni de plus d'un prolongement (3c,9) s'étendant jusque sous les homoplates du patient, qui est également retenu par les tiges de support (3) réalisées dans ce cas en une plus grande longueur correspondante.
